# EUROPEAN PATENT APPLICATION

(11) **EP 4 715 831 A1**
(43) Date of publication of application: **25.03.2026**
(21) Application number: 25175930.4
(22) Date of filing: 13.05.2025
(51) Int. Cl.: G16H 10/60, G16H 30/20

(54) **SYSTEM AND METHOD FOR ANONYMOUS AND SECURE SHARING OF MEDICAL IMAGES THROUGH A MESSAGING TOOL IN MEDICAL IMAGING PICTURE ARCHIVING AND COMMUNICATION SYSTEMS**

(30) Priority: 19.09.2024 US 202418890212
(71) Applicant: FUJIFILM Healthcare Americas Corporation, Lexington, MA 02421 (US)
(72) Inventor: PYRON, Linda Anne, Park Ridge, 60068 (US)
(74) Representative: Marks & Clerk LLP

(57) **Abstract**

A method, system and computer program product for anonymous and secure sharing of medical images through a messaging tool within a picture archiving and communication system (PACS), the PACS comprising medical images captured by a medical imaging apparatus, wherein the medical images include associated digital imaging and communications in medicine (DICOM) data. The method includes: displaying a messaging tool GUI including a dialog thread pane, a message input field, and an icon for capturing a snapshot of a viewer GUI displaying at least one medical image including a DICOM data overlay layer comprising protected health information (PHI) of an imaged subject; capturing the snapshot of the viewer GUI displaying the at least one medical image; generating a de-identified medical image based on the snapshot of the viewer GUI displaying the at least one medical image, wherein the de-identified medical images excludes the PHI.

## Description

### TECHNICAL FIELD

The present invention relates to the field of medical picture archiving and communication systems (PACS); and more particularly relates to a system and method for communicating anonymous and secure information within a PACS.

### BACKGROUND OF THE INVENTION

In the field of medical imaging, the use of PACS has revolutionized the way medical professional's store, access, and analyze patient images. For example, PACS allows healthcare professionals to quickly access and conduct a detailed review of a plurality of medical images in digital format, which can lead to faster and more accurate diagnoses and treatment planning. PACS also allows multiple healthcare professionals to simultaneously access medical images for a particular patient, which provides an efficient way to communicate amongst healthcare professionals and allows for a second opinion in a given medical situation. However, when dealing with sensitive cases involving high-profile individuals that are being evaluated for a health-related issue, situations involving blind-review, or presentations of medical images in an academic setting, the need for anonymity becomes crucial to protect patient privacy. Existing PACS lack an anonymous and secure means of sharing medical images amongst different healthcare professionals, which poses a significant risk to patient confidentiality and health insurance portability and accountability HIPAA laws.

Accordingly, there exists a need for improved methods and communication systems within PACS that enable anonymous and secure sharing of medical images in a messaging tool while ensuring the automatic deletion of sensitive information.

### SUMMARY OF THE INVENTION

It is, therefore, an aspect of the present invention to provide a computer-implemented method for anonymous and secure sharing of at least one medical image through a messaging tool within a PACS, the PACS comprising at least one imaging study including of the at least one medical image captured by a medical imaging apparatus, wherein the at least one medical image includes associated digital imaging and communications in medicine (DICOM) data.

The method includes: displaying, on a workstation display, a first window providing a viewer graphical user interface (GUI), the viewer GUI including at least one pane displaying the at least one medical image of the at least one imaging study of the PACS and a first icon for invoking the messaging tool, wherein the at least one medical image includes a DICOM data overlay layer comprising protected health information (PHI) of an imaged subject; receiving a first user input for invoking the messaging tool; in response to receiving the first user input, displaying, on the workstation display, a second window providing a messaging tool GUI, the messaging tool GUI including an input field for identifying at least one recipient, a dialog thread pane, a message input field, and a second icon for capturing a snapshot of the at least one pane of the viewer GUI displaying the at least one medical image; receiving a second user input for capturing the snapshot; capturing the snapshot of the at least one pane of the viewer GUI displaying the at least one medical image; generating a de-identified medical image based on the snapshot of the at least one pane of the viewer GUI displaying the at least one medical image, wherein the de-identified medical images excludes the PHI; and transmitting and displaying the de-identified medical image in the input field or into the dialog thread pane of the messaging tool GUI.

Another aspect of the present invention a computer system for anonymous and secure sharing of at least one medical image through a messaging tool within a PACS, the PACS comprising at least one imaging study including at least one medical image captured by a medical imaging apparatus, wherein the at least one medical image includes associated DICOM data. The computer system includes one or more computer processors; one or more non-transient, computer-readable storage media; and program instructions stored on the one or more non-transient, computer-readable storage media for execution by at least one of the one or more computer processors.

The program instructions include program instructions to: display, on a workstation display, a first window providing a viewer GUI, the viewer GUI including at least one pane displaying the at least one medical image of the at least one imaging study of the PACS and a first icon for invoking the messaging tool, wherein the at least one medical image includes a DICOM data overlay layer comprising PHI of an imaged subject; receive a first user input for invoking the messaging tool; in response to receiving the first user input, display, on the workstation display, a second window providing a messaging tool GUI, the messaging tool GUI including an input field for identifying at least one recipient, a dialog thread pane, a message input field, and a second icon for capturing a snapshot of the at least one pane of the viewer GUI displaying the at least one medical image; receive a second user input for capturing the snapshot; capture the snapshot of the at least one pane of the viewer GUI displaying the at least one medical image; generate a de-identified medical image based on the snapshot of the at least one pane of the viewer GUI displaying the at least one medical image, wherein the de-identified medical image excludes the PHI; and transmit and display the de-identified medical image in the input field or in the dialog thread pane of the messaging tool GUI.

Another aspect of the present invention provides a computer program product for anonymous and secure sharing of at least one medical image through a messaging tool within a picture archiving and communication system (PACS), the PACS comprising at least one imaging study including the at least one medical image captured by a medical imaging apparatus, wherein the at least one medical image includes associated DICOM data, the computer program product comprising a non-transient computer readable storage medium having program code embodied therewith.

The program code being executable by a processor to: display, on a workstation display, a first window providing a viewer GUI, the viewer GUI including at least one pane displaying the at least one medical image of the at least one imaging study of the PACS and a first icon for invoking the messaging tool, wherein the at least one medical image includes a DICOM data overlay layer comprising PHI of an imaged subject; receive a first user input for invoking the messaging tool; in response to receiving the first user input, display, on the workstation display, a second window providing a messaging tool GUI, the messaging tool GUI including an input field for identifying at least one recipient, a dialog thread pane, a message input field, and a second icon for capturing a snapshot of the at least one pane of the viewer GUI displaying the at least one medical image; receive a second user input for capturing the snapshot; capture the snapshot of the at least one pane of the viewer GUI displaying the at least one medical image; generate a de-identified medical image based on the snapshot of the at least one pane of the viewer GUI displaying the at least one medical image, wherein the de-identified medical image excludes the PHI; and transmit and display the de-identified medical image in the input field or in the dialog thread pane of the messaging tool GUI.

Another aspect of the present invention provides a computer-implemented method for anonymous and secure sharing of at least one medical image through a messaging tool within a PACS, the PACS comprising at least one imaging study including the at least one medical image captured by a medical imaging apparatus, wherein the at least one medical image includes associated DICOM data.

The computer-implemented method includes displaying, on a workstation display, a first window providing a worklist GUI, the worklist GUI including the at least one imaging study including the at least one medical image captured by the medical imaging apparatus and a first icon for invoking a messaging tool; receiving a first user input selecting the at least one imaging study; displaying, on the workstation display, a second window providing a viewer GUI, the viewer GUI including at least one pane displaying the at least one medical image of the selected at least one imaging study and a second icon for invoking the messaging tool, wherein the at least one medical image includes a DICOM data overlay layer comprising PHI of an imaged subject; receiving a second user input selecting the first icon for invoking the messaging tool or the second icon for invoking the messaging tool; in response to receiving the second user input, displaying, on the workstation display, a third window providing a messaging tool GUI of the messaging tool, the messaging tool GUI including an input field for identifying at least one messaging recipient, a dialog thread pane, a message input field, and a third icon for capturing a snapshot of the at least one pane of the viewer GUI displaying the at least one medical image; receiving a third user input selecting the third icon for capturing the snapshot of the at least one pane of the viewer GUI; capturing the snapshot of the at least one pane of the viewer GUI displaying the at least one medical image; generating a de-identified medical image based on the snapshot of the at least one pane of the viewer GUI displaying the at least one medical image, wherein the de-identified medical image excludes the PHI; and transmitting and displaying the de-identified medical image in the input field or into the dialog thread pane of the messaging tool GUI.

Another aspect of the present invention provides a computer-implemented method for anonymous and secure sharing of at least one medical image through a messaging tool within a PACS, the PACS comprising at least one imaging study including the at least one medical image captured by a medical imaging apparatus, wherein the at least one medical image includes associated DICOM data.

The computer-implemented method including displaying, on a workstation display, a first window providing a worklist GUI, the worklist GUI including the at least one imaging study including the at least one medical image captured by the medical imaging apparatus and a first icon for invoking a messaging tool; receiving a first user input selecting the at least one imaging study; displaying, on the workstation display, a second window providing a viewer GUI, the viewer GUI including at least one pane displaying the at least one medical image of the selected at least one imaging study, a second icon for invoking the messaging tool, and a third icon for capturing a snapshot of the at least one pane of the viewer GUI displaying the at least one medical image, wherein the at least one medical image includes a DICOM data overlay layer comprising PHI of an imaged subject; receiving a second user input selecting the first icon for invoking the messaging tool or the second icon for invoking the messaging tool; in response to receiving the second user input, displaying, on the workstation display, a third window providing a messaging tool GUI of the messaging tool, the messaging tool GUI including an input field for identifying at least one messaging recipient, a dialog thread pane, a message input field, and a fourth icon for capturing a snapshot of the at least one pane of the viewer GUI displaying the at least one medical image; receiving a third user input selecting the third icon for capturing the snapshot or the fourth icon for capturing the snapshot; capturing the snapshot of the at least one pane of the viewer GUI displaying the at least one medical image; generating a de-identified medical image based on the snapshot of the at least one pane of the viewer GUI displaying the at least one medical image, wherein the de-identified medical image excludes the PHI; and transmitting and displaying the de-identified medical image in the input field or into the dialog thread pane of the messaging tool GUI.

Additional aspects, advantages and novel features of the present invention will be set forth in part in the description which follows and will in part become apparent to those in the practice of the invention, when considered with the attached figures.

### BRIEF DESCRIPTION OF THE DRAWINGS

The present invention will now be described, by way of example, with reference to the accompanying drawings, in which:
FIG. 1 is a block diagram illustrating an information processing environment for anonymous and secure sharing of medical images through a messaging tool within a PACS in accordance with certain embodiments of the present invention;
FIG. 2 is an exemplary screen shot showing a worklist GUI and a viewer GUI in accordance with certain embodiments of the present invention;
FIG. 3 is a flow chart depicting operational steps for anonymous and secure sharing of medical images through the messaging tool within PACS in accordance with certain embodiments of the present invention;
FIG. 4 is an exemplary screen shot illustrating icons within the worklist GUI and the viewer GUI for invoking the messaging tool in accordance with certain embodiments of the present invention;
FIGS. 5A and 5B are exemplary screen shots illustrating icons within the viewer GUI and the messaging tool GUI for capturing a screenshot of the viewer GUI in accordance with certain embodiments of the present invention;
FIG. 6 illustrates a notification indicating that the snapshot of the viewer GUI is to be pasted into the messaging tool GUI in accordance with certain embodiments of the present invention;
FIGS. 7A and 7B are flow charts depicting operational steps for generating de-identified medical image based on the snapshot;
FIG. 8 is an exemplary screen shot of the messaging tool GUI indicating that an anonymous, de-identified medical image is being shared in accordance with certain embodiments of the present invention; and
FIG. 9 depicts a block diagram of an exemplary computing device that may be used to execute the processes and methods disclosed herein.

Corresponding reference characters indicate corresponding parts throughout the several views. The exemplifications set out herein illustrate currently preferred embodiments of the invention, and such exemplifications are not to be construed as limiting the scope of the invention in any manner.

### DETAILED DESCRIPTION OF THE INVENTION

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer-readable program code/instructions embodied thereon.

Any combination of computer-readable media may be utilized. Computer-readable media may be a computer-readable signal medium or a computer-readable storage medium. A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of a computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain, or store a program for use by or in connection with an instruction execution system, apparatus, or device.

Program code embodied on a computer-readable medium may be transmitted using any appropriate medium, including but not limited to wireless, wireline, optical fiber cable, radio frequency (RF), etc., or any suitable combination of the foregoing.

Computer program code for carrying out operations for aspects of the present invention may be written in any combination of one or more programming languages, including an object-oriented programming language such as Java, Smalltalk, C++ or the like and conventional procedural programming languages, such as the "C" programming language or similar programming languages. The program code may execute entirely on a user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a computing device, such as a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer-implemented process such that the instructions which execute on the computer or other programmable apparatus provide processes for implementing the functions/acts specified in the flowchart and/or block diagram block or blocks.

Medical imaging is the technique and process of capturing images of the interior of a body for clinical analysis and medical intervention, as well as visual representation of the function of some organs or tissues (physiology). Medical imaging seeks to reveal internal structures hidden by the skin and bones, as well as to diagnose and treat disease. Medical imaging also establishes a database of normal anatomy and physiology to make it possible to identify abnormalities. Imaging of removed organs and tissues can also be performed for medical reasons. Medical diagnosis can be performed using medical images obtained by imaging apparatuses such as computed tomography (CT) imaging apparatuses, positron emission tomography (PET) imaging apparatuses, and magnetic resonance imaging (MRI) imaging apparatuses. Whole-Body Maximum Intensity Projection (MIP) imaging can also be used medical diagnosis.

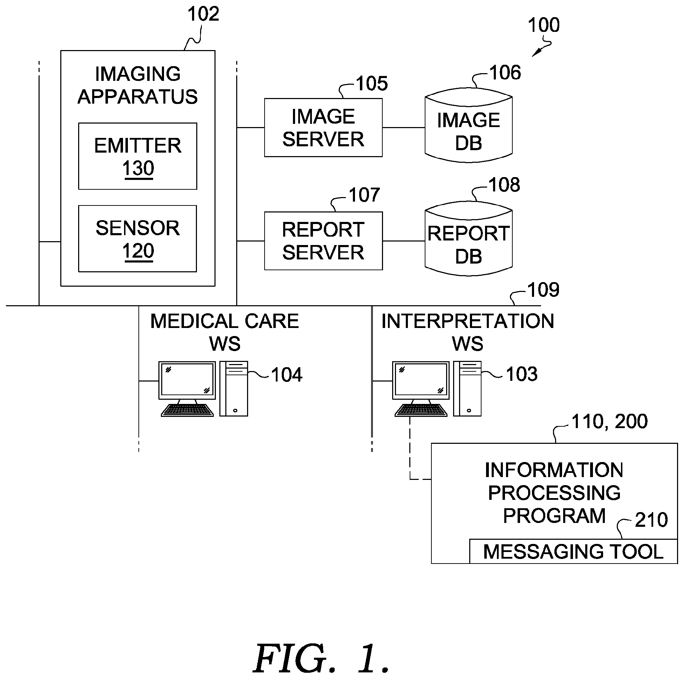

With initial reference to FIG. 1, a block diagram illustrating an information processing environment, generally 100, for capturing, storing, and/or analyzing medical images that can be used in association with an embodiment of the present invention is provided. The information processing environment can include one or more of an imaging apparatus 102, one or more servers (e.g., an image server 105 and a report server 107), and computing devices (e.g., a medical care workstation (WS) 104 and an interpretation WS 103) communicatively coupled together via a network 109. It should be understood that two or more of the aforementioned components may be combined in a single unit. Network 109 can be made up of telecommunication network technologies that are based on physically wired, optical, and/or wireless radio-frequency methods that may be arranged in a variety of network topologies. In general, network 109 can be any set of digital interconnections that allow computing devices to use common communication protocols to communicate with each other.

Imaging apparatus 102 may be one or more imaging devices that include an emitter 130 and a sensor 120. Imaging apparatus 102 is configured to scan the body of a subject by exposing the subject to signals emitted by emitter 130, capturing those signals (or signal reflections) via sensor 120, and thereby obtain detailed internal images of the subject's body (e.g., images of the anatomy and the physiological processes inside the body). The medical images generated by imaging apparatus 102 may be stored in imaging database 106 and/or other database that is communicatively coupled to network 109. For example, imaging apparatus 102 is configured to generate sequential tomographic images of a subject. Imaging apparatus 102 may capture tomographic images along one or more anatomical planes (e.g., the transaxial plane, coronal plane, sagittal plane, median plane, parasagittal plane, and other anatomical planes) of subjects.

Applicable signals emitted by emitter 130 include, but are not limited to, strong magnetic fields, magnetic field gradients, radio waves, X-rays, and ionizing radiation. Imaging apparatus 102 preferably generates heterogeneous sequential tomographic images of the subject. Imaging apparatus 102 can generate medical images using one or more medical imaging techniques that include, but are not limited to, X-ray, CT scan, MRI, PET, MIP, and/or other medical imaging technique capable of generating internal images of a subject's body or part thereof.

The servers may each include a software program providing a database management system (DBMS) installed therein. In some embodiments, image server 105 and report server 107 can be included in one or more computing devices. The servers may include one or more storage media (e.g., flash memory, solid state drive (SSD), or hard disk drive (HDD)) for storing information. For example, image server 105 can include an image database (DB) 106 for at least storing medical images. Report server 107 may include a report DB 108 for at least storing processed reports. Medical care WS 104 can be used by healthcare professionals (e.g., doctors and nurses) to at least observe medical images in detail, view interpretation reports, and/or generate electronic medical records.

Medical care WS 104 may transmit a viewing request for medical images to image server 105, display the received medical images, transmit a viewing request for the associated interpretation report(s) to report server 107, and/or display the received interpretation report. Medical care WS 104 may perform the above processes via executing software programs for respective processes. The steps described in the instant disclosure may be performed by one or more control circuits and/or processors. The interpretation reports can include images analyzed by an information processing program 110 included in interpretation WS 103 (or other computing device communicatively coupled to network 109).

Interpretation WS 103 is a computing device that can be configured to analyze or interpret one or more medical images (e.g., stored in image DB 106 or other datastore communicatively coupled to network 109) as described in the present disclosure. Interpretation WS 103 may be used by user (e.g., a healthcare professional) to interpret one or more medical images and/or create interpretation reports. Interpretation WS 103, or other processor(s) communicatively coupled to network 109, can perform steps defined by an information processing program 110. As will be discussed in more detail below, information processing program 110 is computer code that enables the display, navigation, examination, and/or annotation of sets of medical images. Information processing program 110 can be stored in a database communicatively coupled to the interpretation WS 103. Alternatively, information processing program 110 can be stored in one or more databases that are communicatively coupled to network 109.

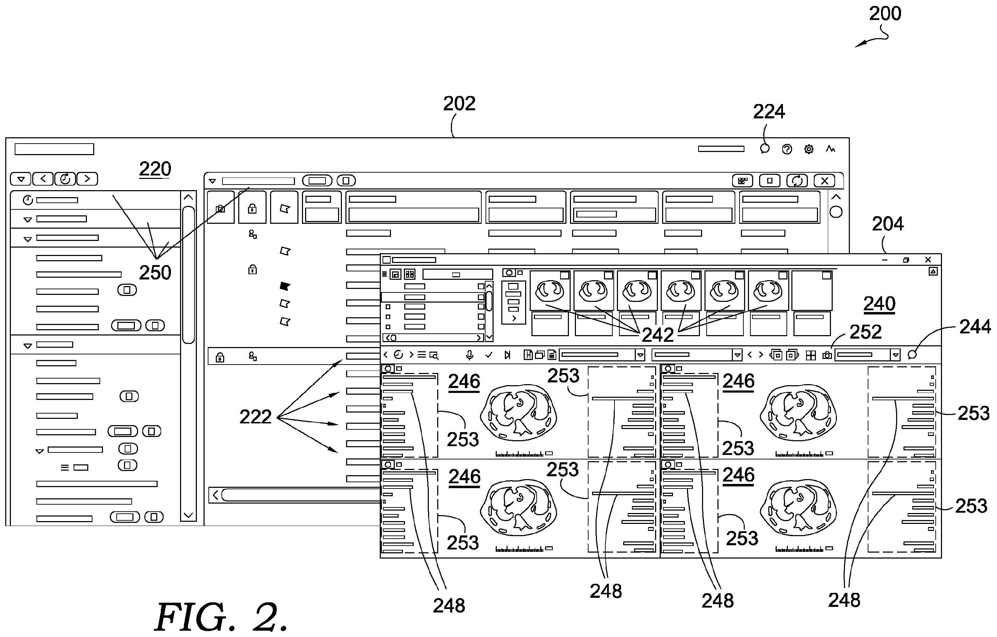

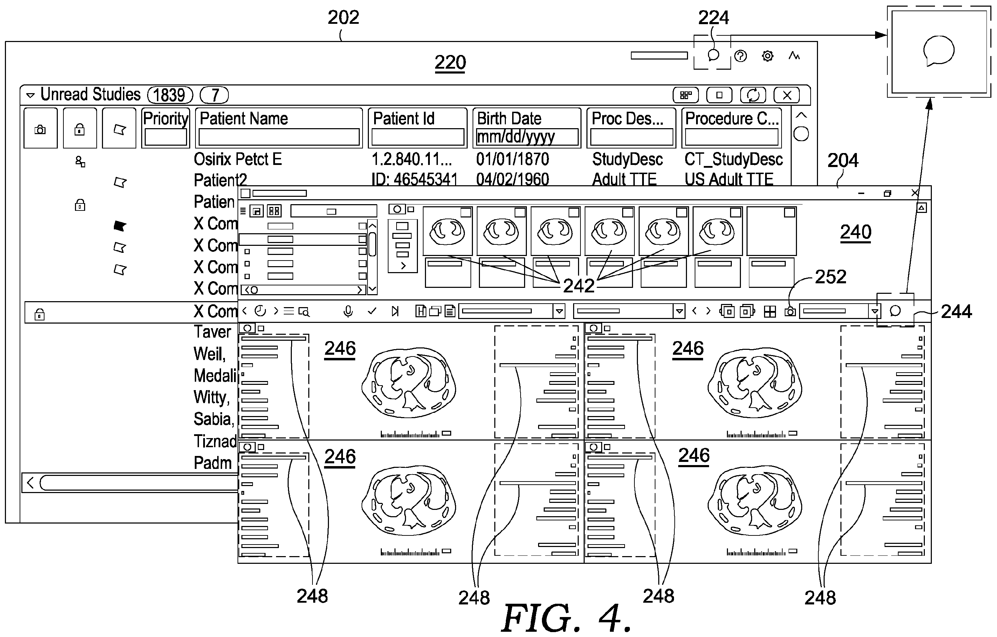

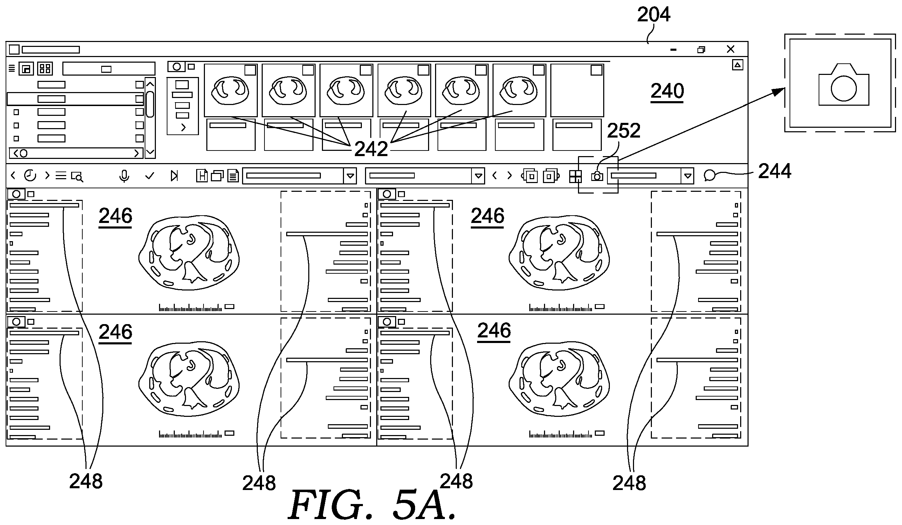

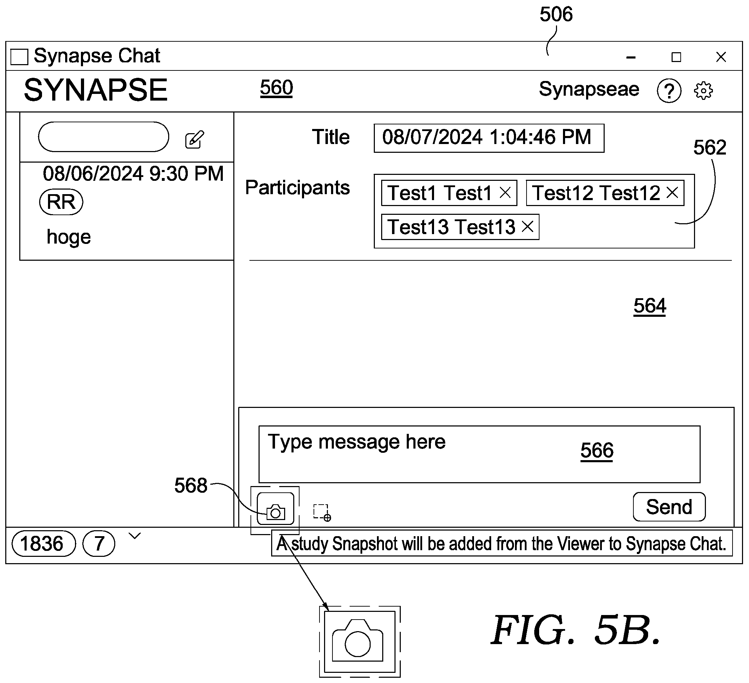

Information processing program 110 may be or may include a picture archiving and communication system (PACS) 200. Referring now to FIGS. 2-5B, embodiments of the present invention provide computer-implemented methods, systems, and computer program products for anonymous and secure sharing of medical images through a messaging tool 210 within PACS 200. PACS 200 includes a plurality of imaging studies including a plurality of medical images captured by one or more medical imaging apparatus 102 and stored in one or more databases 106, 108 that are communicatively coupled to network 109. Each of the plurality of medical images 242 includes corresponding digital imaging and communications in medicine (DICOM) data that comprises a plurality of DICOM data objects corresponding to information entities (IEs). For example, DICOM data objects may correspond to information entities such as a patient, a study, a medical device, a physician's visit, a series of images, a patent schedule, a document, a diagnostic interpretation, etc. DICOM data objects may include attributes consisting of a tag, a value representation describing the data type and format, and an alphanumeric data value. DICOM data may further comprise data objects that constitute protected health information (PHI) 248.

After logging in to PACS 200 on interpretation WS 103 or medical care WS 104, a worklist graphical user interface (GUI) 220 may be provided in a first window 202 displayed on a display of WS 103, 104, at optional step 301. Worklist GUI 220 may include one or more panes 250 displaying one or more lists of a plurality of imaging studies 222. Each of plurality of imaging studies 222 may be associated with one or more medical images 242 of a subject (e.g., person) captured by one or more medical imaging apparatuses 102. First window 202 may also include a first icon 224 for invoking a messaging tool 210 (e.g., a chat tool) which will be described in more detail below.

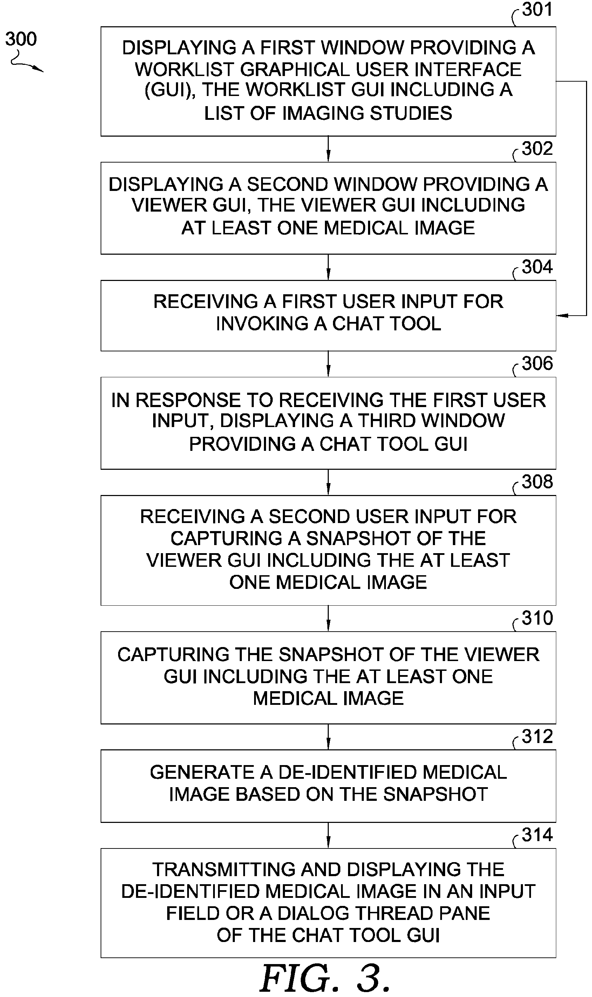

With additional reference to a computer-implemented method 300 shown in FIG. 3, when an imaging study 222 is selected from worklist GUI 220, a second window 204 providing a viewer GUI 240 may be displayed, at step 302, on the display of WS 103, 104. Viewer GUI 240 includes at least one pane 246 that is configured for displaying at least one medical image 242 of the selected at least one imaging study 222. The at least one medical image 242 is displayed in at least one pane 246 for viewing by medical professionals for the purpose of reviewing, evaluating and diagnosing any medical conditions that may be present in the images subject. The at least one medical image 242 includes a DICOM data overlay layer 253 comprising DICOM data including PHI 248 of an imaged subject. PHI 248 may include at least one of the name, associated geographic location smaller than a state, date, telephone number, facsimile number, email address, social security number, medical record number, health plan beneficiary number, account number, certificate or license number, vehicle identifier, device identifier, web uniform resource locator, internet protocol address, biometric identifier, full face photo, or any other uniquely identifying number, characteristic or code of the imaged subject.

In some instances, a medical professional may desire to allow other medical professionals to view the respective at least one medical image 242 for the purpose of obtaining feedback, such as a second opinion, in regard to whether a particular medical condition may be associated with the image subject. To allow for this feedback, viewer GUI 240 may further include a second icon 244 configured for invoking the messaging tool. However, when dealing with sensitive cases involving high-profile individuals that are being evaluated for a health-related issue, situations involving blind-review, or presentations of medical images in an academic setting, the need for anonymity becomes crucial to protect patient privacy. Therefore, certain embodiments of the present invention are configured to allow PHI 248 to be selectively de-identified, such as, but not limited to, suppressed, masked, removed, or otherwise anonymized, from the DICOM data associated with the at least one medical image 242 to be communicated using the messaging tool. PHI 248 may be suppressed when a flag for anonymization for at least one specific field associated with PHI 248 is set and the data in the corresponding field of the DICOM data overlay layer 253 is replaced with one or more special characters. PHI 248 may be masked when a flag for anonymization for at least one specific field associated with PHI 248 is set and the data in the corresponding field of DICOM data overlay layer 253 is hidden by an opaque region of a masking layer overlaid on DICOM data overlay layer 253. PHI 248 may be removed when a flag for anonymization for at least one specific field associated with PHI 248 is set and the data in the corresponding field of DICOM data overlay layer 253 is removed. For example, an attending doctor or radiologist may set a flag for anonymization for at least one specific field associated with PHI 248. As a result, other users with whom the at least one medical image 242 is shared, including doctors and radiologists, are unable to view the flagged fields of PHI 248.

In some embodiments, viewer GUI 240 may include a plurality of panes 246, wherein each pane displays an image of the plurality of medical images 242 of selected imaging study 222. Viewer GUI 240 may further include a first icon 252 for capturing the snapshot of at least one pane 246 of viewer GUI 240 displaying at least one medical image 242.

At step 304, a user input may be received for invoking the messaging tool. For example, the user input for invoking messaging tool 210 may include a user selecting first icon 224 invoking messaging tool 210 on worklist GUI 220 or a user selecting second icon 244 invoking messaging tool 210 on viewer GUI 240.

In response to receiving the user input for invoking the messaging tool, a third window 506 may be displayed on the display of WS 103, 104, at step 306, providing a messaging tool GUI 560 of the messaging tool. Messaging tool GUI 560 may include an input field 562 for at least one other messaging participant, a dialog thread pane 564, an input field 566, and a second icon 568 for capturing a snapshot of at least one pane 246 of viewer GUI 240 displaying at least one medical image 242.

Messaging tool GUI 560 may provide for a search of a list of existing messaging threads and the ability to initiate a new messaging thread. In some embodiments, an existing messaging thread may not provide an input field 562 for specifying an intended messaging participant, as the existing messaging thread is already associated with at least one participant. When a new messaging thread is instantiated, intended participant may be identified via input field 562.

At step 308, a user input may be received for capturing a snapshot of at least one pane 246 of viewer GUI 240. For example, the user input for capturing the snapshot may include selection of a first icon 252 or a second icon 568 for capturing the snapshot of at least one pane 246 of viewer GUI 240 displaying the at least one medical image 242.

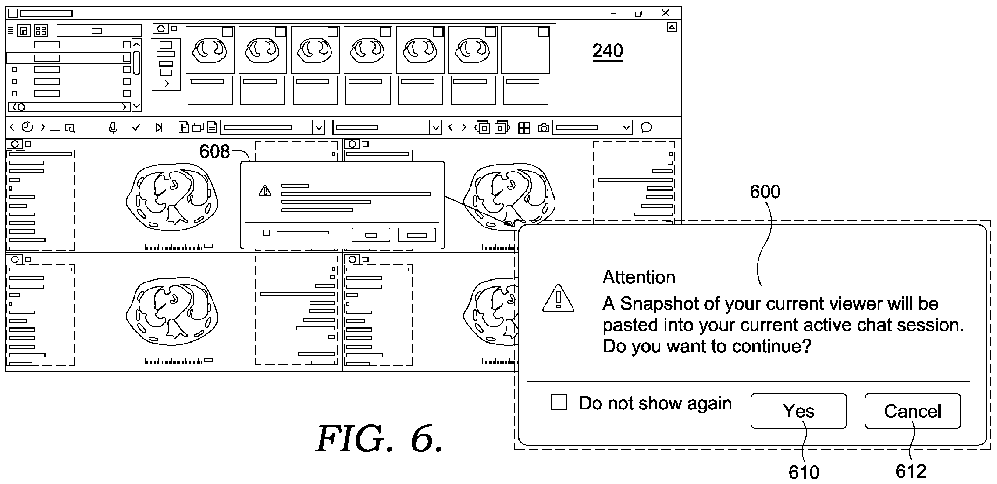

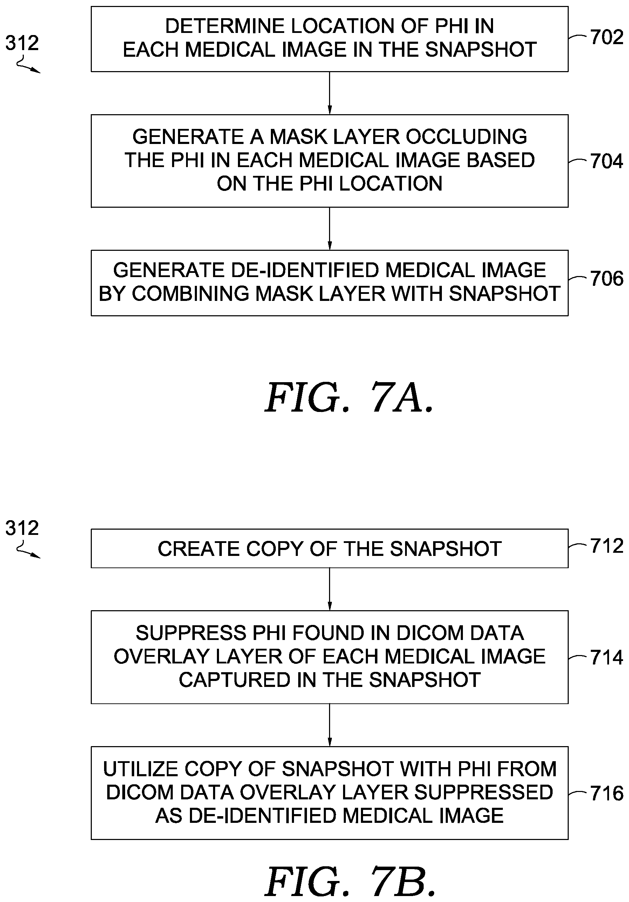

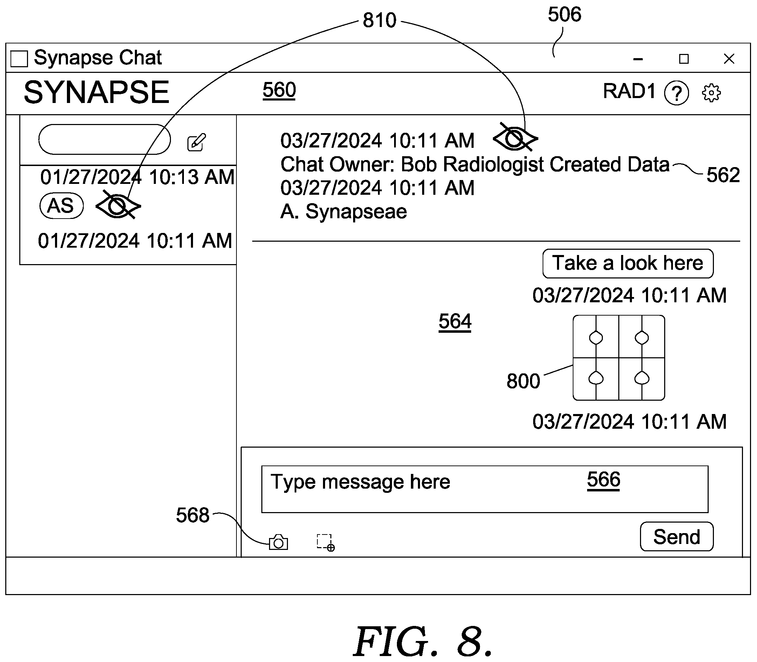

With additional reference to FIGS. 6-8, in response to receiving a user input for capturing the snapshot at step 308, an optional notification 600 indicating that the snapshot of the viewer GUI 240 is to be pasted into the input field or into the dialog thread pane of messaging tool GUI 560 may be displayed. In some embodiments, notification 600 may be presented in a pop-up window 608 overlaying viewer GUI 240. A user input confirming the snapshot of the viewer GUI is to be pasted into the input field or into the dialog thread pane of messaging tool GUI 560 may be displayed. For example, a user input selecting an acknowledgement button 610 may be received. However, if a selection of the of first icon 252 or second icon 568 for capturing the snapshot of at least one pane 246 of viewer GUI 240 was unintended, a user input selecting a cancel or escape button 612 may be received.

In response receiving the user input indicating the snapshot of the viewer GUI is to be pasted into the input field or into the dialog thread pane of the messaging tool GUI may be received, the snapshot of at least one pane 246 of viewer GUI 240 displaying at least one medical image 242 is captured, at step 310. Snapshots may be used to save presentation states for multiple workflow scenarios where a user wants to create a specific display reference. To attach a study snapshot to a messaging thread from viewer GUI 240, an active message may be opened to establish to which message thread the study snapshot is intended.

At step 312, an anonymous, de-identified medical image 800 without PHI is generated based on the snapshot of at least one pane 246 of viewer GUI 240 displaying at least one medical image 242.

For example, in certain embodiments, generating de-identified medical image 800 based on the snapshot may include, determining a location of the PHI in the DICOM data overlay layer 253 in each of the at least one pane displaying the at least one medical image, at step 702. A mask layer may be generated occluding the PHI based on the location of the PHI in each of the at least one pane displaying the at least one medical image, at step 704. The de-identified medical image 800 may be generated by combining the mask layer occluding the PHI with the snapshot.

In other embodiments, generating de-identified medical image 800 based on the snapshot may include, creating a copy of the snapshot of the at least one pane of viewer GUI 240 displaying the at least one medical image, at step 712. The PHI found in the DICOM data overlay layer 253 of each of the at least one medical image captured in the snapshot may be suppressed, at step 714. The copy of the snapshot of the at least one pane of viewer GUI 240 with the PHI from the DICOM data overlay layer 253 suppressed may then be utilized as the de-identified medical image 800.

At step 314, de-identified medical image 800 is displayed in input field 566 or dialog thread pane 564 of messaging tool GUI 560 and transmitted to the at least one other messaging participant. In some embodiments, one or more indicators 810 may be displayed reflecting the use of anonymous, de-identified medical images 800 in messaging tool GUI 560.

Further, in certain embodiments, messaging threads (e.g., chat threads) including anonymous, de-identified medical images 800 may be deleted after being shared. For example, in an attending doctor or radiologist who initiated the messaging thread including anonymous, de-identified medical images 800, may manually delete the anonymized messaging thread. In other embodiments an attending doctor or radiologist who attached anonymous, de-identified medical images 800 to the messaging thread may manually delete the anonymized messaging thread. Additionally, when study and/or examination related to the anonymous, de-identified medical images 800 is completed or when a predetermined time has elapsed after instantiation of the messaging thread, the messaging thread including anonymous, de-identified medical images 800 may be deleted automatically. For example, a messaging thread including anonymous, de-identified medical images 800 may be deleted automatically thirty minutes, an hour, or a day after instantiation of the messaging thread.

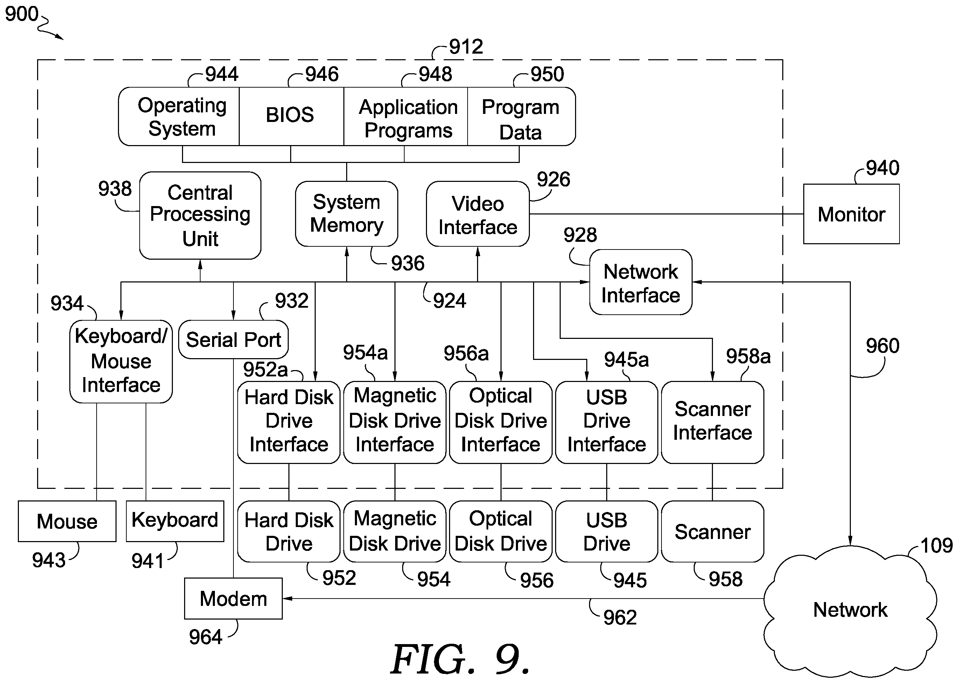

Referring to FIG. 9, an exemplary computing environment 900 is shown that can be utilized through programming to implement any of the processing thus far described. The computing environment 900 may comprise a computer 912 including a system bus 924 that couples a video interface 926, network interface 928, one or more serial ports 932, a keyboard/mouse interface 934, and a system memory 936 to a Central Processing Unit (CPU) 938. Computer 912 may also include a Graphics Processing Unit (GPU) or one or more other special or general purpose processing units. A monitor or display 940 is connected to bus 924 by video interface 926 and provides the user with a graphical user interface to view, edit, and otherwise manipulate items displayed on computer 912. The graphical user interface allows the user to enter commands and information into computer 912 using a keyboard 941 and a user interface selection device 943, such as a mouse or other pointing device. Keyboard 941 and user interface selection device are connected to bus 924 through keyboard/mouse interface 934. The display 940 and user interface selection device 943 are used in combination to form the graphical user interface which may allow a user to implement at least a portion of the present invention. Other peripheral devices may be connected to computer 912 through serial port 932 or universal serial bus (USB) drives 945 to transfer information to and from computer 912.

The system memory 936 is also connected to bus 924 and may include ROM, RAM, an operating system 944, a basic input/output system (BIOS) 946, application programs 948 and program data 950. The computer 912 may further include a hard disk drive 952 for reading from and writing to a hard disk, a magnetic disk drive 954 for reading from and writing to a removable magnetic disk (e.g., floppy disk), and an optical disk drive 956 for reading from and writing to a removable optical disk (e.g., CD ROM or other optical media). The computer 912 may also include USB drives 945 and other types of drives for reading from and writing to flash memory devices (e.g., compact flash, memory stick/PRO and DUO, SD card, multimedia card, smart media card), and a scanner 958 for scanning items such as digital images to be downloaded to computer 912. A hard disk interface 952a, magnetic disk drive interface 954a, an optical drive interface 956a, a USB drive interface 945a, and a scanner interface 958a operate to connect bus 924 to hard disk drive 952, magnetic disk drive 954, optical disk drive 956, USB drive 945 and a scanner 958, respectively. Each of these drive components and their associated computer-readable media may provide computer 912 with non-volatile storage of computer-readable instruction, program modules, data structures, application programs, an operating system, and other data for the computer 912. In addition, it will be understood that computer 912 may also utilize other types of computer-readable media in addition to those types set forth herein, such as digital video disks, random access memory, read only memory, other types of flash memory cards, magnetic cassettes, and the like.

Network interface 928 provides a communication path 960 between bus 924 and network 109, which allows notifications, information and other data to be communicated through network 109 from any of the previously identified devices, and optionally saved in a memory, to the computer 912. This type of logical network connection is commonly used in conjunction with a local area network. Images may also be communicated from bus 924 through a communication path 962 to network 109 using serial port 932 and a modem 964. Using a modem connection between the computer 912 and other computing devices, databases, or the like may be used in conjunction with a wide area network or the Internet. It will be appreciated that the network connections shown herein are merely exemplary, and it is within the scope of the present invention to use other types of network connections between computer 912 and other computing devices including both wired and wireless connections.

As discussed above, embodiments of the present invention provide improved methods and communication systems within PACS that enable anonymous and secure sharing of medical images in a messaging tool while ensuring the automatic deletion of sensitive information.

Based on the foregoing, method, computer system, and program product have been disclosed in accordance with the present invention. However, numerous modifications and substitutions can be made without deviating from the scope of the present invention. Therefore, the present invention has been disclosed by way of example and not limitation.

## Claims

1. A computer system for anonymous and secure sharing of at least one medical image through a messaging tool within a picture archiving and communication system (PACS), the PACS comprising at least one imaging study including at least one medical image captured by a medical imaging apparatus, wherein the at least one medical image includes associated digital imaging and communications in medicine (DICOM) data, the computer system comprising:
one or more computer processors;
one or more non-transient, computer-readable storage media;
program instructions stored on the one or more non-transient, computer-readable storage media for execution by at least one of the one or more computer processors, the program instructions comprising:
program instructions to:
display, on a workstation display, a first window providing a viewer graphical user interface (GUI), the viewer GUI including at least one pane displaying the at least one medical image of the at least one imaging study of the PACS and a first icon for invoking the messaging tool, wherein the at least one medical image includes a DICOM data overlay layer comprising protected health information (PHI) of an imaged subject;
receive a first user input for invoking the messaging tool;
in response to receiving the first user input, display, on the workstation display, a second window providing a messaging tool GUI, the messaging tool GUI including an input field for identifying at least one recipient, a dialog thread pane, a message input field, and a second icon for capturing a snapshot of the at least one pane of the viewer GUI displaying the at least one medical image;
receive a second user input for capturing the snapshot;
capture the snapshot of the at least one pane of the viewer GUI displaying the at least one medical image;
generate a de-identified medical image based on the snapshot of the at least one pane of the viewer GUI displaying the at least one medical image, wherein the de-identified medical image excludes the PHI; and
transmit and display the de-identified medical image in the input field or in the dialog thread pane of the messaging tool GUI.

2. The computer system of claim 1, wherein the PHI includes at least one of names, associated geographic locations smaller than a state, dates, telephone number, facsimile numbers, email address, social security numbers, medical record numbers, health plan beneficiary numbers, account numbers, certificate or license numbers, vehicle identifiers, device identifiers, web uniform resource locators, internet protocol addresses, biometric identifiers, full face photos, or any other uniquely identifying number, characteristic or code.

3. The computer system of claim 1, wherein the program instructions further comprise program instructions to:
in response to receiving second user input for capturing the snapshot, display a notification indicating that the snapshot of the viewer GUI is to be pasted into the input field or into the dialog thread pane of the messaging tool GUI;
receive a third user input confirming the snapshot of the viewer GUI is to be pasted into the input field or into the dialog thread pane of the messaging tool GUI; and
in response to receiving the third user input, capture the snapshot of the at least one pane of the viewer GUI displaying the at least one medical image.

4. The computer system of claim 1, wherein the viewer GUI further comprises a third icon for capturing the snapshot of the at least one pane of the viewer GUI displaying the at least one medical image; and
wherein the program instructions to receive the second user input for capturing the snapshot comprise program instructions to: receive the second user input selecting the second icon for capturing the snapshot of the at least one pane of the viewer GUI or the third icon for capturing the snapshot of the at least one pane of the viewer GUI.

5. The computer system of claim 1, wherein the program instructions further comprise program instructions to:
display, on the workstation display, a third window providing a worklist GUI, the worklist GUI including the at least one imaging study including the at least one medical image captured by the medical imaging apparatus and a third icon for invoking the messaging tool;
receive another user input selecting at one of the at least one imaging study; and
in response to receiving the other user input, select the one of the at least one imaging study, displaying, on the workstation display, the first window providing the viewer GUI.

6. The computer system of claim 5, wherein the program instructions to receive the first user input for invoking the messaging tool comprise program instructions to receive the first user input selecting the first icon for invoking the messaging tool or the third icon for invoking the messaging tool.

7. The computer system of claim 1, wherein the program instructions to generate the de-identified medical image based on the snapshot comprise program instructions to:
determine a location of the PHI in each of the at least one pane displaying the at least one medical image;
generate a mask layer occluding the PHI in each of the at least one pane displaying the at least one medical image; and
generate the de-identified medical image by combining the mask layer occluding the PHI with the snapshot.

8. The computer system of claim 1, wherein the program instructions to generate the de-identified medical image based on the snapshot comprise program instructions to:
create a copy of the snapshot of the at least one pane of the viewer GUI displaying the at least one medical image;
suppress the PHI found in a DICOM data overlay layer of each of the at least one medical image captured in the snapshot; and
utilize the copy of the snapshot of the at least one pane of the viewer GUI with the PHI from the DICOM data overlay layer removed as the de-identified medical image.

9. A computer program product for anonymous and secure sharing of at least one medical image through a messaging tool within a picture archiving and communication system (PACS), the PACS comprising at least one imaging study including the at least one medical image captured by a medical imaging apparatus, wherein the at least one medical image includes associated digital imaging and communications in medicine (DICOM) data, the computer program product comprising a non-transient computer readable storage medium having program code embodied therewith, the program code executable by a processor to:
display, on a workstation display, a first window providing a viewer graphical user interface (GUI), the viewer GUI including at least one pane displaying the at least one medical image of the at least one imaging study of the PACS and a first icon for invoking the messaging tool, wherein the at least one medical image includes a DICOM data overlay layer comprising protected health information (PHI) of an imaged subject;
receive a first user input for invoking the messaging tool;
in response to receiving the first user input, display, on the workstation display, a second window providing a messaging tool GUI, the messaging tool GUI including an input field for identifying at least one recipient, a dialog thread pane, a message input field, and a second icon for capturing a snapshot of the at least one pane of the viewer GUI displaying the at least one medical image;
receive a second user input for capturing the snapshot;
capture the snapshot of the at least one pane of the viewer GUI displaying the at least one medical image;
generate a de-identified medical image based on the snapshot of the at least one pane of the viewer GUI displaying the at least one medical image, wherein the de-identified medical image excludes the PHI; and
transmit and display the de-identified medical image in the input field or in the dialog thread pane of the messaging tool GUI.

10. The computer program product of claim 9, wherein the computer program product further comprises program code to:
in response to receiving second user input for capturing the snapshot, display a notification indicating that the snapshot of the viewer GUI is to be pasted into the input field or into the dialog thread pane of the messaging tool GUI;
receive a third user input confirming the snapshot of the viewer GUI is to be pasted into the input field or into the dialog thread pane of the messaging tool GUI; and
in response to receiving the third user input, capture the snapshot of the at least one pane of the viewer GUI displaying the at least one medical image.

11. The computer program product of claim 9, wherein the viewer GUI further comprises a third icon for capturing the snapshot of the at least one pane of the viewer GUI displaying the at least one medical image; and
wherein the program code to receive the second user input for capturing the snapshot comprises program code to: receive the second user input selecting the second icon for capturing the snapshot of the at least one pane of the viewer GUI or the third icon for capturing the snapshot of the at least one pane of the viewer GUI.

12. The computer program product of claim 9, wherein the program code further comprises program code to;
display, on the workstation display, a third window providing a worklist GUI, the worklist GUI including the at least one imaging study including the at least one medical image captured by the medical imaging apparatus and a third icon for invoking the messaging tool;
receive another user input selecting one of the at least one imaging study; and
in response to receiving the other user input, select the one or the at least one imaging study, displaying, on the workstation display, the first window providing the viewer GUI.

13. The computer program product of claim 12, wherein the program code to receive the first user input for invoking the messaging tool comprises program code to receive the first user input selecting the first icon for invoking the messaging tool or the third icon for invoking the messaging tool.

14. The computer program product of claim 9, wherein the program code to generate the de-identified medical image based on the snapshot comprise program code to:
determine a location of the PHI in each of the at least one pane displaying the at least one medical image;
generate a mask layer occluding the PHI in each of the at least one pane displaying the at least one medical image; and
generate the de-identified medical image by combining the mask layer occluding the PHI with the snapshot.

15. The computer program product of claim 9, wherein the program code to generate the de-identified medical image based on the snapshot comprise program code to:
create a copy of the snapshot of the at least one pane of the viewer GUI displaying the at least one medical image;
suppress the PHI found in the DICOM data overlay layer of each of the at least one medical image captured in the snapshot; and
utilize the copy of the snapshot of the at least one pane of the viewer GUI with the PHI from the DICOM data overlay layer removed as the de-identified medical image.
